# DEMANDE DE BREVET EUROPEEN

(11) **EP 1 275 644 A1**
(43) Date de publication de la demande: **15.01.2003**
(21) Numéro de dépôt: 02291746.2
(22) Date de dépôt: 11.07.2002
(51) Int. Cl.: C07D 209/14, C07D 417/12, C07D 401/06, C07D 413/14, C07D 417/14, C07D 409/14, A61K 31/405, A61P 9/00

(54) **Nouveaux dérivés de benzène sulfonamide, leur procédé de préparation et les compositions pharmaceutiques qui les contiennent**

(30) Priorité: 13.07.2001 FR 0109338
(71) Demandeur: Les Laboratoires Servier, 92415 Courbevoie Cedex (FR)
(72) Inventeur: Lavielle, Gilbert, 78170 La Celle Saint Cloud (FR); Cimetiere, Bernard, 75020 Paris (FR); Verbeuren, Tony, 78540 Vernouillet (FR); Simonet, Serge, 78700 Conflans Sainte Honorine (FR); Vayssettes-Courchay, Christine, 91430 Igny (FR)

(57) **Abrégé**

Composé de formule (I) : 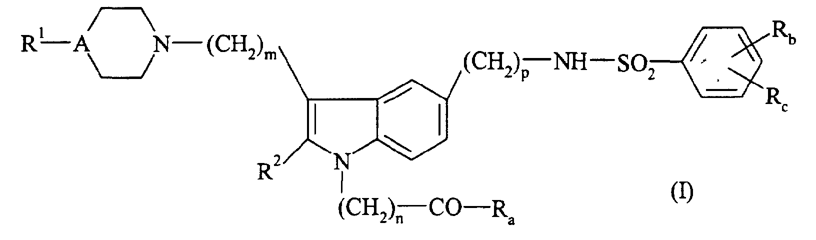 dans laquelle :
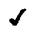 Rₐ représente un groupement hydroxy, alkoxy, aryloxy ou arylalkyloxy
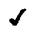 A représente un groupement CH ou un atome d'azote et dans ce cas R¹ est tel que définit dans la description
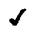 ou R¹-A représente ensemble un atome d'oxygène ou un groupement 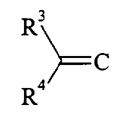 dans lequel R³, R⁴ sont tels que définis dans la description
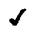 R² étant un atome d'hydrogène ou un groupement alkyle
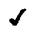 R_{b}, R_{c}, identiques ou différents, représentent un atome d'hydrogène, un atome d'halogène, un groupement alkyle, un groupement alkoxy, un groupement hydroxy ou un groupement trihalogénoalkyle,
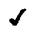 n est un entier compris inclusivement entre 2 et 6
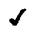 m et p sont des entiers compris inclusivement entre 0 et 6

## Description

La présente invention concerne des nouveaux dérivés de benzènesulfonamide, leur procédé de préparation et les compositions pharmaceutiques qui les contiennent.

Des composés possédant un enchaînement benzènesulfonamide ont été décrits dans la demande EP 864561 pour leur caractère donneur de NO et antagonistes des récepteurs au thromboxane A₂ (TXA₂), ainsi que dans la demande EP 648741 pour leurs seules propriétés antagonistes des récepteurs du TXA₂ ou WO 9506046 en tant qu'antagonistes des récepteurs du TXA₂ et de leurs précurseurs, la prostaglandine H₂ (PGH₂).

Les composés de la présente invention possèdent une structure originale qui leur confère un caractère antagoniste des récepteurs du TXA₂ et antagoniste des récepteurs sérotoninergiques 5HT₂.

L'agrégation plaquettaire et les vasospasmes jouent un rôle essentiel dans l'étiologie et le développement des maladies cardiovasculaires athéro-thrombotiques. Le TXA₂, métabolite de l'acide arachidonique, et la sérotonine (5HT), neurotransmetteur, sont tous deux de puissants agents vasoconstricteurs, et peuvent induire ou renforcer l'activation des plaquettes, conduisant à leur agrégation. Les actions vasoconstrictrices et proagrégantes du TXA₂ se font par l'intermédiaire de récepteurs membranaires appelé TP-récepteurs (Medicinal Research Reviews, 1991, 11, 5, p.503) alors que celles de la sérotonine se font par l'intermédiaire des récepteurs 5HT₁ ou 5HT₂ (T.I.P.S., 1991, 121, p.223). Les stratégies de recherche mises en oeuvre afin de trouver des agents qui bloquent la production et/ou l'activation du TXA₂ ont conduit au développement d'antagonistes sélectifs des récepteurs TP, d'inhibiteurs de TXA₂-synthase, ou d'agents mixtes possédant les deux propriétés (Medicinal Research Reviews, ibd., T.I.P.S., 1991, 121, 158). La sérotonine agit, tout comme le TXA₂, en stimulant les plaquettes et les constrictions vasculaires, et son activité se trouve renforcée dans les maladies athéro-thrombotiques.

La conception de composés s'opposant à la fois au processus faisant intervenir le thromboxane et à celui faisant intervenir la sérotonine, est d'une grande utilité pour les cliniciens. De tels produits présentent l'avantage d'offrir une protection plus complète, à la fois contre l'activation des plaquettes et contre les vasospasmes. Ils pourront donc être utiles pour le traitement des pathologies liées à une activité exagérée de TXA₂ et de 5-HT en particulier dans le traitement des maladies cardiovasculaires athéro-thrombotiques telles que l'infarctus du myocarde, l'angine de poitrine, les accidents vasculaires cérébraux, la maladie de Raynaud, ou encore l'asthme, les bronchospasmes, mais aussi la migraine et les maladies veineuses.

La présente invention concerne les composés de formule (I): 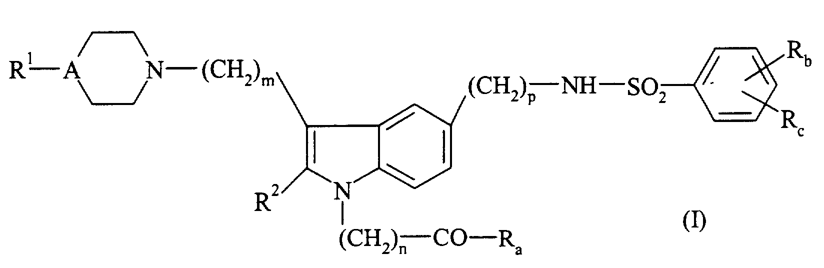 dans laquelle :
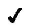 Rₐ représente un groupement hydroxy, alkoxy, aryloxy éventuellement substitué ou arylalkyloxy éventuellement substitué, amino, alkylamino, dialkylamino, arylamino éventuellement substitué, arylalkylamino éventuellement substitué,
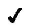 A représente :
   - soit un groupement CH et dans ce cas R¹ représente un atome d'hydrogène ou un groupement alkyle, cycloalkyle, cycloalkylalkyle, aryle éventuellement substitué, arylcarbonyle éventuellement substitué, arylcarbonylalkyle éventuellement substitué, aryloxy éventuellement substitué, aryloxyalkyle éventuellement substitué, arylthio éventuellement substitué, arylthioalkyle éventuellement substitué, arylamino éventuellement substitué, arylalkylamino éventuellement substitué, hétéroaryle éventuellement substitué, hétéroarylalkyle éventuellement substitué, hétéroarylcarbonyle éventuellement substitué, hétéroarylcarbonylalkyle éventuellement substitué, hétéroaryloxy éventuellement substitué, hétéroaryloxyalkyle éventuellement substitué, hétéroarylthio éventuellement substitué, hétéroarylthioalkyle éventuellement substitué, hétéroarylamino éventuellement substitué ou hétéroarylalkylamino éventuellement substitué,
   - soit un atome d'azote et dans ce cas R¹ représente un atome d'hydrogène ou un groupement alkyle, cycloalkyle, cycloalkylalkyle, aryle éventuellement substitué, arylcarbonyle éventuellement substitué, arylcarbonylalkyle éventuellement substitué, arylsulfonyle éventuellement substitué, aryloxyalkyle éventuellement substitué, arylthioalkyle éventuellement substitué, hétéroaryle éventuellement substitué, hétéroarylalkyle éventuellement substitué, hétéroarylcarbonyle éventuellement substitué, hétéroarylcarbonylalkyle éventuellement substitué, hétéroaryloxyalkyle éventuellement substitué, hétéroarylsulfonyle éventuellement substitué ou hétéroarylthioalkyle éventuellement substitué,
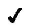 ou R¹-A représentent ensemble un atome d'oxygène ou un groupement 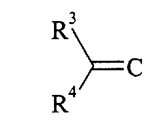 dans lequel R³, R⁴ identiques ou différents représentent un atome d'hydrogène, un groupement aryle éventuellement substitué, alkyle ou un hétéroaryle éventuellement substitué,
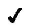 R² étant un atome d'hydrogène ou un groupement alkyle,
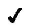 R_{b}, R_{c}, identiques ou différents, représentent un atome d'hydrogène, un atome d'halogène, un groupement alkyle, un groupement alkoxy, un groupement hydroxy ou un groupement trihalogénoalkyle,
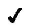 n est un entier compris inclusivement entre 2 et 6
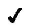 m et p sont des entiers, identiques ou différents, compris inclusivement entre 0 et 6
leurs énantiomères, diastéréoisomères ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkoxy désigne un groupement alkyle-oxy, contenant de 1 à 6 atomes de carbone, linéaire ou ramifié,
- le terme trihalogénoalkyle désigne un chaîne carbonée, contenant de 1 à 3 atomes de carbone et de 1 à 3, identiques ou différents, atomes d'halogène,
- le terme cycloalkyle désigne un groupement cyclique saturé contenant de 3 à 8 atomes de carbone,
- le terme aryle désigne un groupement phényle ou naphtyle,
- le terme hétéroaryle désigne un groupement monocyclique aromatique, ou bicyclique dans lequel au moins un des cycles est aromatique, contenant de 5 à 11 chaînons, et 1 à 5 hétéroatomes choisis parmi azote, oxygène et soufre,
- le terme "substitué" associé aux expressions aryle, arylcarbonyle, arylcarbonylalkyle, aryloxy, aryloxyalkyle, arylthio, arylthioalkyle, arylamino, arylalkylamino, hétéroaryle, hétéroarylalkyle, hétéroarylcarbonyle, hétéroarylcarbonylalkyle, hétéroaryloxy, hétéroaryloxyalkyle, hétéroarylthio, hétéroarylthioalkyle, hétéroarylamino, et hétéroarylalkylamino, arylsulfonyle, arylsulfonylalkyle, hétéroarylsulfonyle, hétéroarylsulfonylalkyle, signifie que les groupements concernés sont substitués sur la partie aromatique par un ou deux substituants, identiques ou différents, choisis parmi les atomes d'halogène et les groupements alkyle, alkoxy, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que les groupements hétéroaryle et hétéroarylalkyle peuvent être en plus substitués par un groupement oxo sur la partie non-aromatique de l'hétéroaryle.

Des composés préférés de l'invention sont ceux pour lesquels, pris ensemble ou séparément, la valeur du substituant m vaut 2, la valeur de n vaut 2, la valeur de p vaut 2, le substituant Rₐ représente un groupement hydroxy, et le substituant R² représente un atome d'hydrogène ou un groupement méthyle.

Un aspect particulièrement avantageux de l'invention concerne les composés de formule (I) pour lesquels m, n et p valent chacun 2, Rₐ représente un groupement hydroxy, R² représente un atome d'hydrogène ou un groupement méthyle, R_{b} représente un atome d'halogène, R_{c} représente un atome d'hydrogène, et A représente un atome d'azote et dans ce cas R¹ représente un atome d'hydrogène, un groupement alkyle, cycloalkyle, aryle éventuellement substitué, arylcarbonyle éventuellement substitué, hétéroaryle éventuellement substitué, ou bien A représente un groupement CH et dans ce cas R¹ représente un atome d'hydrogène, un groupement alkyle, cycloalkyle, aryle éventuellement substitué, arylcarbonyle éventuellement substitué ou hétéroaryle éventuellement substitué, ou bien R¹-A représente ensemble un atome d'oxygène ou un groupement R³R⁴C=C dans lequel R³ et R⁴ représentent un groupement aryle éventuellement substitué.

Plus particulièrement les composés préférés de formule (I) sont ceux pour lesquels m, n et p valent chacun 2, Rₐ représente un groupement hydroxy, R² représente un atome d'hydrogène ou un groupement méthyle, R_{b} représente un atome d'halogène en position *para* du cycle phényle, R_{c} représente un atome d'hydrogène, et
- soit A représente un atome d'azote et dans ce cas R¹ représente un groupement phényle éventuellement substitué par un atome d'halogène, ou hétéroaryle à 9 chaînons comportant un à deux hétéroatomes choisis parmi l'atome d'azote, d'oxygène et de soufre, et éventuellement substitué par un atome d'halogène,
- soit A représente un groupement CH et dans ce cas R¹ représente un groupement phényle éventuellement substitué par un atome d'halogène, phénylcarbonyle éventuellement substitué par un atome d'halogène, ou hétéroaryle à 9 chaînons comportant un à deux hétéroatomes choisis parmi l'atome d'azote, d'oxygène et de soufre, et éventuellement substitué par un atome d'halogène,
- soit R¹-A représente ensemble un groupement R³R⁴C=C dans lequel R³ et R⁴ représentent chacun un groupement phényle éventuellement substitué par un atome d'halogène.

De façon avantageuse l'invention concerne les composés de formule (I) pour lesquels le groupement R¹ hétéroaryle éventuellement substitué concerne un groupement benzisoxazolyle éventuellement substitué par un atome d'halogène, benzothiènyle éventuellement substitué par un atome d'halogène, ou benzisothiazolyle éventuellement substitué par un atome d'halogène.

Parmi les composés préférés de l'invention, on peut citer l'acide 3-[3-{2-[4-(1,2-benzisothiazol-3-yl)-1-pipérazinyl]éthyl}-5-(2-{[(4-chlorophényl)sulfonyl]amino}éthyl)-1*H*-indol-1-yl]propanoïque.

La présente invention concerne également le procédé de préparation des composés de formule (I) caractérisé en ce que l'on utilise comme produit de départ un composé de formule (II) : 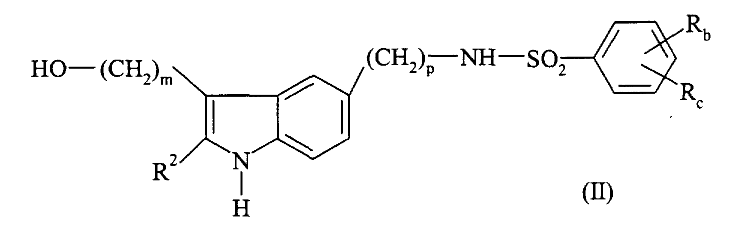 dans laquelle R², R_{b}, R_{c}, m et p ont la même signification que dans la formule (I),
- qui est halogéné pour conduire au composé de formule de formule (III) : 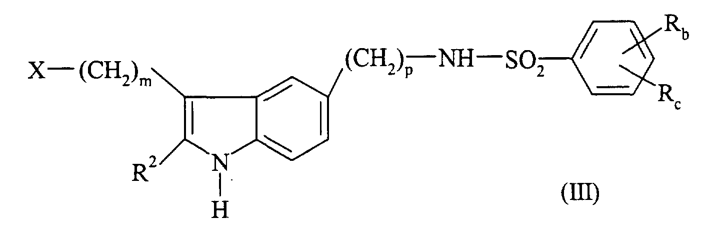 dans laquelle R², R_{b}, R_{c}, m et p ont la même signification que dans la formule (I), X représente un atome d'halogène,
- dont l'atome d'halogène est substitué par un groupement amino de formule : 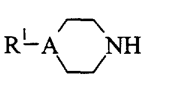 pour conduire au composé de formule (IV): 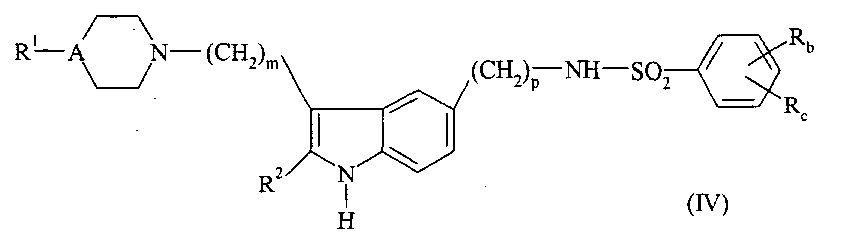 dans laquelle R¹, A, R², R_{b,} R_{c,} m et p ont la même signification que dans la formule (I),
- qui subit une condensation sur l'azote indolique avec de l'acrylonitrile, suivi d'une hydrolyse du dérivé nitrile en milieu alcalin pour conduire à un composé de formule (I/a), cas particulier de la formule (I): 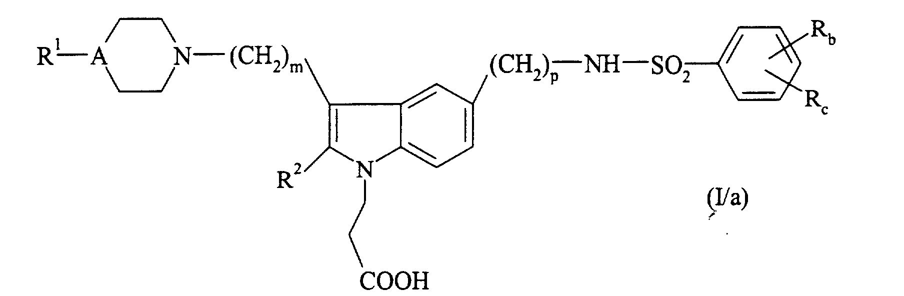 dans laquelle R¹, A, R², R_{b,} R_{c,} m et p ont la même signification que dans la formule (I),
- dont la fonction acide carboxylique est éventuellement transformée par réduction en aldéhyde, pour réagir avec un ylure de phosphore approprié, et qui après une réduction catalytique conduit à (I/b), cas particulier de la formule (I): 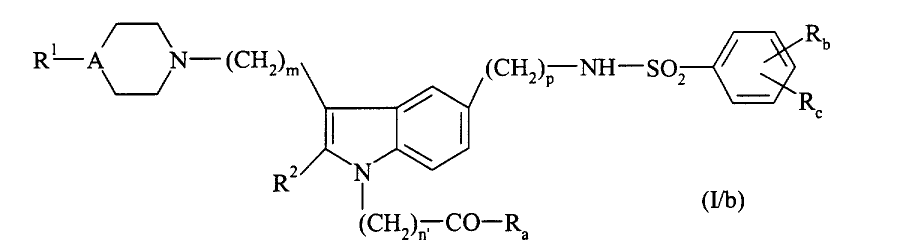 dans laquelle R¹, A, R², Rₐ, R_{b}, R_{c}, m et p ont la même signification que dans la formule (I), et n' est un entier compris entre 4 et 6,
composés de formule (I/b) qui peuvent être soumis à une hydrolyse de la fonction ester, en milieu basique ou acide selon les groupements réactifs sur la molécule, pour conduire au composé de formule (I/c): 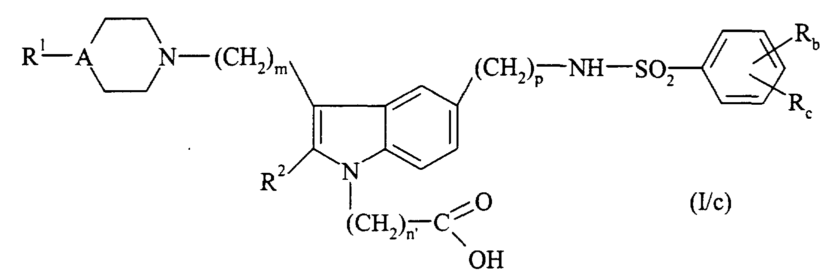 cas particulier des composés de fomule (I) dans laquelle R¹, R², R_{b}, R_{c}, m et p sont tels que définis dans la formule (I), et n' est un entier compris entre 4 et 6,
composés (I/a), (I/b) et (I/c) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable,
étant entendu qu'à tout moment jugé opportun au cours du procédé précédemment décrit, la fonction acide carboxylique peut être estérifiée, ou bien la fonction ester carboxylique peut être hydrolysée en acide correspondant, ce dernier pouvant être à nouveau transformé en un autre ester pour les besoins de la synthèse.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention et ne la limitent en aucune façon. Les structures des composés décrits ont été confirmés par les techniques spectroscopiques et spectrométriques usuelles.

Les produits de départ utilisés sont des produits connus ou préparés selon des modes opératoires connus.

### Préparation A : 4-chloro-N-{2-[3-(2-hydroxyéthyl)-1H-indol-5-yl]éthyl} benzènesulfonamide

### Stade a : N-[2-(4-aminophényl)éthyl]-4-chlorobenzènesulfonamide

A une solution de 46,5 g (340 mmol) de 4-(2-aminoéthyl)aniline dans un litre de dichlorométhane sont ajoutés à température ambiante 47,5 ml de triéthylamine, puis par portion 72 g de chlorure de 4-chlorobenzènesulfonyle. Le mélange est alors agité une nuit, puis filtré. Le solide obtenu est lavé à l'eau, et séché au dessiccateur pour donner le produit attendu.

### Stade b : Hydrate de 4-chloro-N-[2-(4-hydrazinophényl)éthyl]benzènesulfonamide

A une suspension de 20 g (64 mmol) du produit décrit au stade précédent, dans 140 ml d'acide chlorhydrique concentré est ajoutée à -10°C une solution de 11,5 g de nitrite de sodium dans 40 ml d'eau. Après agitation 10 minutes à -10°C est ajoutée une solution de 200 g de dichlorure d'étain dans 260 ml d'acide chlorhydrique concentré. La suspension est agitée 3 heures à température ambiante. Le solide obtenu est filtré et repris dans 800 ml de méthanol. Les insolubles sont filtrés, et le volume de méthanol est réduit par évaporation jusqu'à un volume de 400 ml. Le produit attendu est isolé par cristallisation.

### Stade c : 4-Chloro-N-{2-[3-(2-hydroxyéthyl)-1H-indol-5-yl]éthyl} benzènesulfonamide

A une solution de 15 g (41 mmol) du produit décrit au stade précédent, dans un mélange de 145 ml d'éthanol et 15 ml d'eau est ajoutée à 60°C en 20 minutes une solution de 5,8 ml (45 mmol) de 2-éthoxytétrahydrofurane dans 240 ml d'éthanol. Le mélange est chauffé à reflux pendant 4 heures. Après évaporation du solvant et purification sur colonne de silice avec comme éluant un mélange dichlorométhane/méthanol/ammoniaque (95/5/0,5), on obtient le produit attendu.

### Préparation B : 4-chloro-N-{2-[3-(2-hydroxyéthyl)-2-méthyl-1H-indol-5-yl]éthyl} benzènesulfonamide

Le produit attendu est obtenu selon les procédés décrits dans la Préparation A, en remplaçant au Stade c, le 2-éthoxytétrahydrofurane par la 5-hydroxy-2-pentanone.

### Préparation C : 4-Chlore-N-{2-[3-(3-hydroxypropyl)-1H-indol-5-yl]éthyl} benzènesulfonamide

### Stade a : N-[2-(4-aminophényl)éthyl]-4-chlorobenzènesulfonamide

Le produit attendu est obtenu selon le procédé décrit dans la Préparation A, Stade a.

### Stade b : N-[2-(4-amino-3-iodophényl)éthyl]-4-chlorobenzènesulfonamide

A une solution de 5 g (16 mmol) du produit précédemment synthétisé, dans 100 ml de méthanol et 50 ml de dichlorométhane sont ajoutés à température ambiante 2,08 g de carbonate de calcium et 5,56 g de dichloroiodate de benzyltriméthylammonium. Après 3 heures d'agitation, la suspension est filtrée et le filtrat est évaporé. Le solide obtenu est repris dans le dichlorométhane, la solution est lavée avec une solution aqueuse à 10 % de bisulfate de sodium, puis à l'eau, et ensuite séchée sur sulfate de magnésium. L'évaporation du solvant conduit au produit attendu.

### Stade c : 4-Chloro-N-{2-[3-(3-hydroxypropyl)-1H-indol-5-yl]éthyl} benzènesulfonamide

Préparation de l'éther de triméthylsilyl 5-(triméthylsilyl)-4-pentynyle.
A une solution de 20 g (237 mmol) de 4-pentyn-1-ol dans 250 ml de THF à -35°C sont ajoutés 300 ml d'une solution 1,6 N de n-butyllithium dans l'hexane. Après 30 minutes d'agitation à -20°C, 62 ml (486 mmol) de chlorure de triméthylsilyle sont ajoutés. Après remontée à température ambiante et agitation 2 heures, 600 ml d'éther et 600 ml de pentane sont ajoutés, suivis d'une solution aqueuse à 1 % en carbonate de sodium. Après décantation, séchage de la phase organique sur sulfate de magnésium, et évaporation des solvants le dérivé sylilé est isolé.

Dans 100 ml de DMF sont mis en suspension 5 g (11,5 mmol) de l'éther de triméthylsilyl 5-(triméthylsilyl)-4-pentynyle précédemment préparé, 2,6 g (11,5 mmol) du produit décrit dans la Préparation C, Stade b, 6 g de carbonate de sodium, et 100 mg d'acétate de palladium. Le Mélange réactionnel est chauffé à 110°C pendant 4 heures. Le DMF est alors évaporée, et le mélange est repris dans le dichlorométhane. La phase organique est lavée à l'eau, séchée sur sulfate de magnésium, et le solvant est évaporé. L'huile noire obtenue est reprise avec 50 ml d'éthanol, puis traitée par 5 ml d'acide chlorhydrique 1 N. Après 2 heures à température ambiante, l'éthanol est évaporé. L'huile est reprise avec du dichlorométhane, la phase organique lavée à l'eau, séchée sur sulfate de magnésium, et évaporé pour conduire au produit attendu.

### EXEMPLE 1 : Acide 3-(5-(2-{[(4-chlorophényl)sulfonyl]amino}éthyl)-3-{2-[4-(4-fluorobenzoyl)-1-pipéridinyl)éthyl}-1H-indol-1-yl)propanoïque

### Stade a : N-{2-[3-(2-bromoéthyl)-1H-indol-5-yl]éthyl}-4-chlorobenzènesulfonamide

A une suspension de 4 g (10,5 mmol) du produit obtenu dans la Préparation A, stade c, dans 60 ml d'acétonitrile sont ajoutés à température ambiante 3,3 g (12,6 mmol) de triphénylphosphine, puis 4,2 g (12,6 mmol) de tétrabromure de carbone dans 30 ml d'acétonitrile. Le mélange est agité 2 heures, puis le solvant est évaporé. Après une chromatographie sur colonne de silice en utilisant comme éluant un mélange acétate d'éthyle/cyclohexane (20/80), on obtient le produit attendu.

### Stade b : 4-Chloro-N-[2-(3-{2-[4-(4-fluorobenzoyl)-1-pipéridinyl]éthyl}-1H-indol-5-yl)éthyl]benzènesulfonamide

A une solution de 3,2 g (0,72 mmol) du produit obtenu au stade précédent, dans 100 ml de DMF est ajouté 11 g (2,9 mmol) de carbonate de potassium. Le mélange est porté à 70°C sous azote pendant 1 heure. Le DMF est alors évaporée et l'huile résultante est reprise dans du dichlorométhane. La phase organique obtenue est lavée à l'eau, séchée sur sulfate de magnésium et évaporée. Le résidu est purifié par chromatographie sur colonne de silice en utilisant comme éluant un mélange dichlorométhane/méthanol (47/3).

### Stade c : 4-Chloro-N-[2-(1-(2-cyanoéthyl)-3-{2-[4-(4-fluorobenzoyl)-1-pipéridinyl] éthyl}-1H-indol-5-yl)éthyl]benzènesulfonamide

A une solution de 2,4 g (4,22 mmol) du produit obtenu au stade précédent dans 40 ml de DMF, sont ajoutés à température ambiante 190 mg d'hydrure de sodium à 60 %. Après la fin du dégagement gazeux, une solution de 450 mg d'acrylonitrile dans 10 ml de DMF est ajoutée à température ambiante. Après 1 heure d'agitation, 50 ml d'une solution aqueuse saturée en chlorure de sodium est ajoutée. Après extraction à l'acétate d'éthyle, séchage sur sulfate de magnésium et évaporation des solvants, le produit attendu est purifié par une chromatographie sur colonne de silice en utilisant comme éluant un mélange dichlorométhane/méthanol (98/2).

### Stade d : Acide 3-(5-(2-{[(4-chlorophényl)sulfonyl]amino}éthyl)-3-{2-[4-(4-fluorobenzoyl)-1-pipéridinyl]éthyl}-1H-indol-1-yl)propanoïque

A une solution de 1,4 g (2,25 mmol) du produit obtenu au stade précédent dans 100 ml d'isopropanol est ajouté 20 ml d'une solution aqueuse de potasse à 10 %. Après 4 heures de reflux, l'isopropanol est évaporé, et de l'eau est ajoutée. L'ajout d'acide acétique jusqu'à pH=5 provoque la séparation d'une huile, qui est purifiée par chromatographie sur colonne de silice avec comme éluant un mélange dichlorométhane/méthanol/acide acétique (95/5/0,5) pour conduire au produit attendu.

| *Microanalyse élémentaire :* | | | | | |
|---|---|---|---|---|---|
| | C | H | N | Halogène | S |
| % Trouvé | 61,13 | 5,45 | 6,39 | 5,77 | 4,49 |
| % Calculé | 61,92 | 5,51 | 6,56 | 5,54 | 5,01 |

### EXEMPLE 2 : Acide 3-(5-(2-{[(4-chlorophényl)sulfonyl]amino}éthyl)-3-{2-[4-(4-fluorophényl)-1-pipérazinyl]éthyl}-1H-indol-1-yl)propanoïque

Le produit attendu est obtenu selon le procédé décrit dans l'Exemple 1, en remplaçant, au Stade b, le tosylate de 4-(4-fluorobenzoyl)pipéridinium par la 1-(4-fluorophényl) pipérazine.

| *Microanalyse élémentaire :* | | | | | |
|---|---|---|---|---|---|
| | C | H | N | Halogène | S |
| % Trouvé | 60,17 | 5,51 | 8,77 | 6,05 | 5,1 |
| % Calculé | 60,73 | 5,59 | 9,14 | 5,78 | 5,23 |

### EXEMPLE 3 : Acide 3-[3-(2-{4-[bis(4-fluorophényl)méthylène]-1-pipéridinyl} éthyl)-5-(2-{[(4-chlorophényl)sulfonyl]amino}éthyl)-1H-indol-1-yl] propanoïque

Le produit attendu est obtenu selon le procédé décrit dans l'Exemple 1, en remplaçant au Stade b, le tosylate de 4-(4-fluorobenzoyl)pipéridinium par la 4-[bis(4-fluorophényl) méthylène]pipéridine.

| *Microanalyse élémentaire :* | | | | | |
|---|---|---|---|---|---|
| | C | H | N | Cl | S |
| % Trouvé | 64,90 | 5,34 | 5,79 | 5,03 | 4,36 |
| % Calculé | 65,22 | 5,33 | 5,85 | 4,94 | 4,46 |

### EXEMPLE 4 : Acide 3-(5-(2-{[(4-chlorophényl)sulfonyl]amino}éthyl)-3-{2-[4-(6-fluoro-1,2-benzisoxazol-3-yl)-1-pipéridinyl]éthyl}-1H-indol-1-yl) propanoïque

Le produit attendu est obtenu selon le procédé décrit dans l'Exemple 1, en remplaçant au Stade b, le tosylate de 4-(4-fluorobenzoyl)pipéridinium par le chlorhydrate de 6-fluoro-3-(4-pipéridinyl)-1,2-benzisoxazole.

| *Microanalyse élémentaire :* | | | | | |
|---|---|---|---|---|---|
| | C | H | N | Cl | S |
| % Trouvé | 60,62 | 5,17 | 8,44 | 5,72 | 4,90 |
| % Calculé | 60,68 | 5,25 | 8,58 | 5,43 | 4,91 |

### EXEMPLE 5 : Acide 3-(5-(2-{[(4-chlorophényl)sulfonyl]amino}éthyl)-3-{2-[4-(6-fluoro-1,2-benzisothiazol-3-yl)-1-pipéridinyl]éthyl}-1H-indol-1-yl) propanoïque

Le produit attendu est obtenu selon le procédé décrit dans l'Exemple 1, en remplaçant au Stade b, le tosylate de 4-(4-fluorobenzoyl)pipéridinium par le chlorhydrate de 6-fluoro-3-(4-pipéridinyl)-1,2-benzisothiazole.

| *Microanalyse élémentaire :* | | | | | |
|---|---|---|---|---|---|
| | C | H | N | Halogène | S |
| % Trouvé | 58,75 | 5,12 | 8,14 | 5,49 | 9,25 |
| % Calculé | 59,23 | 5,12 | 8,37 | 5,30 | 9,58 |

### EXEMPLE 6 : Acide 3-[3-{2-[4-(1,2-benzisothiazol-3-yl)-1-pipérazinyl]éthyl}-5-(2-{[(4-chlorophényl)sulfonyl]amino}éthyl)-1H-indol-1-yl]propanoïque

Le produit attendu est obtenu selon le procédé décrit dans l'Exemple 1, en remplaçant au Stade b, le tosylate de 4-(4-fluorobenzoyl)pipéridinium par le chlorhydrate 3-(1-pipérazinyl)-1,2-benzisothiazole.

| *Microanalyse élémentaire :* | | | | | |
|---|---|---|---|---|---|
| | C | H | N | Halogène | S |
| % Trouvé | 58,28 | 5,20 | 10,29 | 5,65 | 9,88 |
| % Calculé | 58,93 | 5,25 | 10,74 | 5,44 | 9,83 |

### EXEMPLE 7 : Acide 3-(5-(2-{[(4-chlorophényl)sulfonyl]amino}éthyl)-3-{2-[4-(6-fluoro-1-benzothièn-2-yl)-1-pipéridinyl]éthyl}-1H-indol-1-yl) propanoïque

Le produit attendu est obtenu selon le procédé décrit dans l'Exemple 1, en remplaçant au Stade b, le tosylate de 4-(4-fluorobenzoyl)pipéridinium par le chlorhydrate de 4-(6-fluoro-1-benzothièn-2-yl)pipéridine.

| *Microanalyse élémentaire :* | | | | | |
|---|---|---|---|---|---|
| | C | H | N | Cl | S |
| % Trouvé | 64,90 | 5,34 | 5,79 | 5,03 | 4,36 |
| % Calculé | 65,22 | 5,33 | 5,85 | 4,94 | 4,46 |

### EXEMPLE 8 : Acide 3-(5-(2-{[(4-chlorophényl)sulfonyl]amino}éthyl)-3-{2-[4-(6-fluoro-1,2-benzisoxazol-3-yl)-1-pipéridinyl]éthyl}-2-méthyl-1H-indol-1-yl)propanoïque

### Stade a : N-{2-[3-(2-bromoéthyl)-2-méthyl-1H-indol-5-yl]éthyl}-4-chlorobenzènesulfonamide

Le produit attendu est obtenu par bromation du réactif synthétisé dans la Préparation B, Stade c, et selon le procédé décrit dans l'Exemple 1, Stade a.

### Stade b : 4-Chloro-N-[2-(3-(2-[4-(6-fluoro-1,2-benzisoxazol-3-yl)-1-pipéridinyl] éthyl}-2-méthyl-1H-indol-5-yl)éthyl]benzènesulfonamide

Le produit attendu est obtenu à partir du réactif préparé au stade précédent et selon le procédé décrit dans l'Exemple 4, Stade b.

### Stade c : 4-Chloro-N-[2-(1-(2-cyanoéthyl)-3-{2-[4-(6-fluoro-1,2-benzisoxazol-3-yl)-1-piperidinyl]éthyl}-2-méthyl-1H-indol-5-yl)éthyl] benzènesulfonamide

Le produit attendu est obtenu à partir du réactif préparé au stade précédent et selon le procédé décrit dans l'Exemple 1, Stade c.

### Stade d : Acide 3-(5-(2-{[(4-chlorophényl)sulfonyl]amino}éthyl)-3-{2-[4-(6-fluoro -1,2-benzisoxazol-3-yl)-1-pipéridinyl]éthyl}-2-méthyl-1H-indol-1-yl) propanoïque

Le produit attendu est obtenu à partir du réactif préparé précédemment et selon le procédé décrit dans l'Exemple 1, Stade d.

| *Microanalyse élémentaire :* | | | | | |
|---|---|---|---|---|---|
| | C | H | N | Cl | S |
| % Trouvé | 61,31 | 5,43 | 8,47 | 5,64 | 4,51 |
| % Calculé | 61,21 | 5,44 | 8,40 | 5,31 | 4,81 |

### EXEMPLE 9 : Acide 3-(5-(2-{[(4-chlorophényl)sulfonyl]amino}éthyl)-3-{3-[4-(4-fluorobenzoyl)-1-pipéridinyl]propyl}-1H-indol-1-yl)propanoïque

### Stade a : N-{2-[3-(3-bromopropyl)-lH-indol-5-yl]éthyl}-4-chlorobenzènesulfonamide

Le produit attendu est obtenu à partir du réactif préparé dans la Préparation C, Stade c et selon le procédé décrit dans l'exemple 1, stade a.

### Stade b : 4-Chloro-N-[2-(3-{3-[4-(4-fluorobenzoyl)-1-pipéridinyl]propyl}-1H-indol-5-yl)éthyl]benzènesulfonamide

Le produit attendu est obtenu à partir du réactif préparé au stade précédent et selon le procédé décrit dans l'Exemple 1, Stade b.

### Stade c : 4-Chloro-N-[2-(1-(2-cyanoéthyl)-3-{3-[4-(4-fluorobenzoyl)-1-pipéridinyl]propyl]-1H-indol-5-yl)éthyl]benzenesulfonamide

Le produit attendu est obtenu à partir du réactif préparé au stade précédent et selon le procédé décrit dans l'Exemple 1, Stade c.

### Stade d : Acide 3-(5-(2-{[(4-chlorophényl)sulfonyl]amino}éthyl)-3-{3-[4-(4-fluorobenzoyl)-1-pipéridinyl]propyl}-1H-indol-1-yl)propanoïque

Le produit attendu est obtenu à partir du réactif préparé au stade précédent et selon le procédé décrit dans l'Exemple 1, Stade d.

| *Microanalyse élémentaire :* | | | | | |
|---|---|---|---|---|---|
| | C | H | N | Cl | S |
| % Trouvé | 62,14 | 5,59 | 6,37 | 6,12 | 4,19 |
| % Calculé | 62,42 | 5,70 | 6,42 | 5,42 | 4,90 |

### EXEMPLE 10 : Chlorydrate de l'acide 3-(5-(2-{[(4-chlorophényl)sulfonyl]amino} éthyl)-3-{3-[4-(4-fluorophényl)-1-pipérazinyl]propyl}-1H-indol-1-yl)propanoïque

Le produit attendu est obtenu selon les procédé décrit dans l'Exemple 9, en remplaçant au Stade b le tosylate de 4-(4-fluorobenzoyl)pipéridinium par le 1-(4-fluorophényl)pipérazine.

| *Microanalyse élémentaire :* | | | | | |
|---|---|---|---|---|---|
| | C | H | N | Halogène | S |
| % Trouvé | 57,80 | 5,89 | 8,07 | 4,82 | 4,59 |
| % Calculé | 57,92 | 5,62 | 8,44 | 5,34 | 4,83 |

### ETUDE PHARMACOLOGIQUE

### EXEMPLE A : Agrégation plaquettaire chez l'homme

Le sang veineux est obtenu de volontaires humains n'ayant pas pris de l'aspirine pendant au moins 14 jours précédent l'expérience. Le sang est prélevé sur citrate de sodium (0.109 M) (1 vol. de citrate sur 9 vol. de sang). Le plasma riche en plaquettes (PRP) est obtenu après centrifugation (20°C) à 200 g pendant 10 minutes. Le nombre de plaquettes est en moyenne de 250000 PL/mm³. Le PRP est conservé à la température de la pièce jusqu'au moment du test et est utilisé dans les 2 heures qui suivent le prélèvement. L'agoniste TXA₂, le U46619 est utilisé à la concentration de 1 µM et la 5-hydroxytryptamine à la concentration de 10 µM, ce dernier en présence d'adénosine diphosphate à 0.3 µM et d'adrénaline à 1 µM.

Les composés de l'invention inhibent l'agrégation plaquettaire induite par l'agoniste TXA₂ ainsi que celle produite par la 5-hydroxytryptamine. A titre d'exemple les IC₅₀ du composé de l'exemple 6 sur les deux expériences sont respectivement de 1,5 µM et de 3,0 µM.

Les valeurs indiquent que les composés de l'invention sont de puissants anti-agrégants plaquettaires agissant de façon balancée sur les deux voies d'activation, celle du TXA₂ et celle de la sérotonine.

### EXEMPLE B : Composition pharmaceutique

| Formule de préparation pour 1000 comprimés dosés à 5 mg : | |
|---|---|
| Composé de l'exemple 4 | 5 g |
| Hydroxypropylméthylcellulose | 2 g |
| Amidon de blé | 10 g |
| Lactose | 100 g |
| Stéarate de magnésium | 3 g |

## Revendications

1. Composés de formule (I): 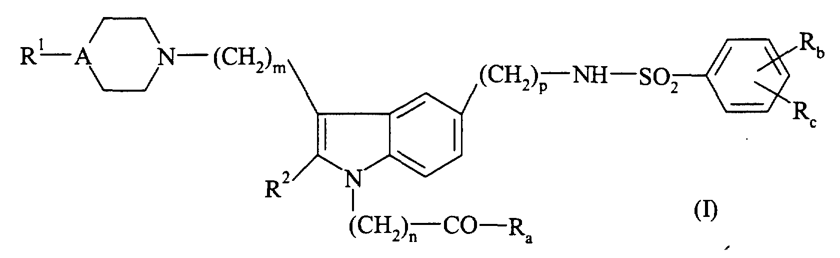 dans laquelle :
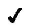 Rₐ représente un groupement hydroxy, alkoxy, aryloxy éventuellement substitué ou arylalkyloxy éventuellement substitué, amino, alkylamino, dialkylamino, arylamino éventuellement substitué, arylalkylamino éventuellement substitué,
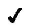 A représente :
• soit un groupement CH et dans ce cas R¹ représente un atome d'hydrogène ou un groupement alkyle, cycloalkyle, cycloalkylalkyle, aryle éventuellement substitué, arylcarbonyle éventuellement substitué, arylcarbonylalkyle éventuellement substitué, aryloxy éventuellement substitué, aryloxyalkyle éventuellement substitué, arylthio éventuellement substitué, arylthioalkyle éventuellement substitué, arylamino éventuellement substitué, arylalkylamino éventuellement substitué, hétéroaryle éventuellement substitué, hétéroarylalkyle éventuellement substitué, hétéroarylcarbonyle éventuellement substitué, hétéroarylcarbonylalkyle éventuellement substitué, hétéroaryloxy éventuellement substitué, hétéroaryloxyalkyle éventuellement substitué, hétéroarylthio éventuellement substitué, hétéroarylthioalkyle éventuellement substitué, hétéroarylamino éventuellement substitué ou hétéroarylalkylamino éventuellement substitué,
• soit un atome d'azote et dans ce cas R¹ représente un atome d'hydrogène ou un groupement alkyle, cycloalkyle, cycloalkylalkyle, aryle éventuellement substitué, arylcarbonyle éventuellement substitué, arylcarbonylalkyle éventuellement substitué, arylsulfonyle éventuellement substitué, aryloxyalkyle éventuellement substitué, arylthioalkyle éventuellement substitué, hétéroaryle éventuellement substitué, hétéroarylalkyle éventuellement substitué, hétéroarylcarbonyle éventuellement substitué, hétéroarylcarbonylalkyle éventuellement substitué, hétéroaryloxyalkyle éventuellement substitué, hétéroarylsulfonyle éventuellement substitué ou hétéroarylthioalkyle éventuellement substitué,
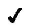 ou R¹-A représentent ensemble un atome d'oxygène ou un groupement 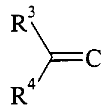 dans lequel R³, R⁴ identiques ou différents représentent un atome d'hydrogène, un groupement aryle éventuellement substitué, alkyle ou un hétéroaryle éventuellement substitué,
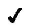 R² étant un atome d'hydrogène ou un groupement alkyle,
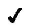 R_{b}, R_{c}, identiques ou différents, représentent un atome d'hydrogène, un atome d'halogène, un groupement alkyle, un groupement alkoxy, un groupement hydroxy ou un groupement trihalogénoalkyle,
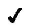 n est un entier compris inclusivement entre 2 et 6
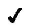 m et p sont des entiers, identiques ou différents, compris inclusivement entre 0 et 6
leurs énantiomères, diastéréoisomères ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkoxy désigne un groupement alkyle-oxy, contenant de 1 à 6 atomes de carbone, linéaire ou ramifié,
- le terme trihalogénoalkyle désigne un chaîne carbonée, contenant de 1 à 3 atomes de carbone et de 1 à 3, identiques ou différents, atomes d'halogène,
- le terme cycloalkyle désigne un groupement cyclique saturé contenant de 3 à 8 atomes de carbone,
- le terme aryle désigne un groupement phényle ou naphtyle,
- le terme hétéroaryle désigne un groupement monocyclique aromatique, ou bicyclique dans lequel au moins un des cycles est aromatique, contenant de 5 à 11 chaînons, et 1 à 5 hétéroatomes choisis parmi azote, oxygène et soufre,
- le terme "substitué" associé aux expressions aryle, arylcarbonyle, arylcarbonylalkyle, aryloxy, aryloxyalkyle, arylthio, arylthioalkyle, arylamino, arylalkylamino, hétéroaryle, hétéroarylalkyle, hétéroarylcarbonyle, hétéroarylcarbonylalkyle, hétéroaryloxy, hétéroaryloxyalkyle, hétéroarylthio, hétéroarylthioalkyle, hétéroarylamino, et hétéroarylalkylamino, arylsulfonyle, arylsulfonylalkyle, hétéroarylsulfonyle, hétéroarylsulfonylalkyle, signifie que les groupements concernés sont substitués sur la partie aromatique par un ou deux substituants, identiques ou différents, choisis parmi les atomes d'halogène et les groupements alkyle, alkoxy, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que les groupements hétéroaryle et hétéroarylalkyle peuvent être en plus substitués par un groupement oxo sur la partie non-aromatique de l'hétéroaryle.

2. Composés de formule (I) selon la revendication 1 pour lesquels m vaut 2, leurs énantiomères, diastéréoisomères ainsi que leurs sels d'addition à un acide ou à une base pharmaceutiquement acceptable.

3. Composés de formule (I) selon une quelconque des revendications 1 ou 2 pour lesquels n vaut 2, leurs énantiomères, diastéréoisomères ainsi que leurs sels d'addition à un acide ou à une base pharmaceutiquement acceptable.

4. Composés de formule (I) selon une quelconque des revendications 1 à 3 pour lesquels p vaut 2, leurs énantiomères, diastéréoisomères ainsi que leurs sels d'addition à un acide ou à une base pharmaceutiquement acceptable.

5. Composés de formule (I) selon une quelconque des revendications 1 à 4 pour lesquels Rₐ représente un groupement hydroxy, leurs énantiomères, diastéréoisomères ainsi que leurs sels d'addition à un acide ou à une base pharmaceutiquement acceptable.

6. Composés de formule (I) selon une quelconque des revendications 1 à 5 pour lesquels R² représente un atome d'hydrogène ou un groupement méthyle, leurs énantiomères, diastéréoisomères ainsi que leurs sels d'addition à un acide ou à une base pharmaceutiquement acceptable.

7. Composés de formule (I) selon une quelconque des revendications 1 à 6 pour lesquels m, n ou p valent chacun 2, Rₐ représente un groupement hydroxy, R² représente un atome d'hydrogène ou un groupement méthyle, R_{b} représente un atome d'halogène, R_{c} représente un atome d'hydrogène, et A représente un atome d'azote et dans ce cas R¹ représente un atome d'hydrogène, un groupement alkyle, cycloalkyle, aryle éventuellement substitué, arylcarbonyle éventuellement substitué, hétéroaryle éventuellement substitué, ou bien A représente un groupement CH et dans ce cas R¹ représente un atome d'hydrogène, un groupement alkyle, cycloalkyle, aryle éventuellement substitué, arylcarbonyle éventuellement substitué ou hétéroaryle éventuellement substitué, ou bien R¹-A représente ensemble un atome d'oxygène ou un groupement R³R⁴C=C dans lequel R³ et R⁴ représentent un groupement aryle éventuellement substitué, leurs énantiomères, diastéréoisomères ainsi que leurs sels d'addition à un acide ou à une base pharmaceutiquement acceptable.

8. Composés de formule (I) selon une quelconque des revendications 1 à 7 pour lesquels m, n et p valent chacun 2, Rₐ représente un groupement hydroxy, R² représente un atome d'hydrogène ou un groupement méthyle, R_{b} représente un atome d'halogène en position *para* du cycle phényle, R_{c} représente un atome d'hydrogène, et
- soit A représente un atome d'azote et dans ce cas R¹ représente un groupement phényle éventuellement substitué par un atome d'halogène, ou hétéroaryle à 9 chaînons comportant un à deux hétéroatomes choisis parmi l'atome d'azote, d'oxygène et de soufre, et éventuellement substitué par un atome d'halogène,
- soit A représente un groupement CH et dans ce cas R¹ représente un groupement phényle éventuellement substitué par un atome d'halogène, phénylcarbonyle éventuellement substitué par un atome d'halogène, ou hétéroaryle à 9 chaînons comportant un à deux hétéroatomes choisis parmi l'atome d'azote, d'oxygène et de soufre, et éventuellement substitué par un atome d'halogène,
- soit R¹-A représente ensemble un groupement R³R⁴C=C dans lequel R³ et R⁴ représentent chacun un groupement phényle éventuellement substitué par un atome d'halogène,
leurs énantiomères, diastéréoisomères ainsi que leurs sels d'addition à un acide ou à une base pharmaceutiquement acceptable.

9. Composés de formule (I) selon une quelconque des revendications 1 à 8 pour lesquels le groupement R¹ hétéroaryle éventuellement substitué concerne un groupement benzisoxazolyle éventuellement substitué par un atome d'halogène, benzothiènyle éventuellement substitué par un atome d'halogène, ou benzisothiazolyle éventuellement substitué par un atome d'halogène, leurs énantiomères, diastéréoisomères ainsi que leurs sels d'addition à un acide ou à une base pharmaceutiquement acceptable.

10. Composé de formule (I) selon une quelconque des revendications 1 à 9 qui est l'acide 3-[3-{2-[4-(1,2-benzisothiazol-3-yl)-1-pipérazinyl]éthyl}-5-(2-{[(4-chlorophényl)sulfonyl]amino}éthyl)-1*H*-indol-1-yl]propanoïque

11. Procédé de préparation des composés de formule (I) selon la revendication 1 **caractérisé en ce que** l'on utilise comme produit de départ un composé de formule (II) : 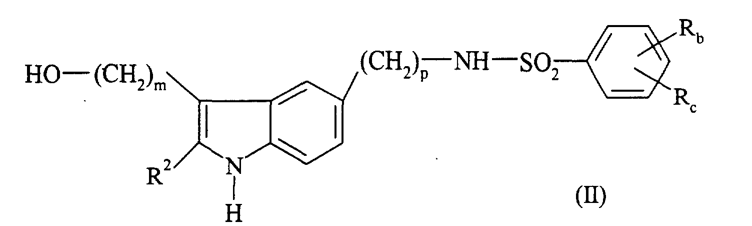 dans laquelle R², R_{b}, R_{c}, m et p ont la même signification que dans la formule (I),
- qui est halogéné pour conduire au composé de formule de formule (III) : 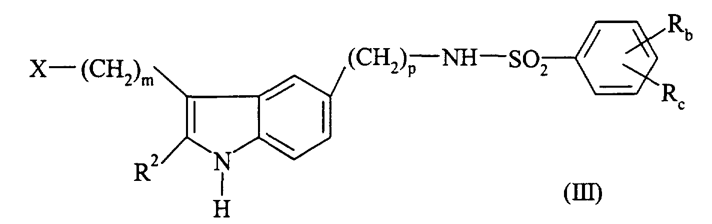 dans laquelle R², R_{b}, R_{c}, m et p ont la même signification que dans la formule (I), X représente un atome d'halogène,
- dont l'atome d'halogène est substitué par un groupement amino de formule : 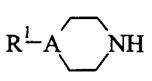 pour conduire au composé de formule (IV): 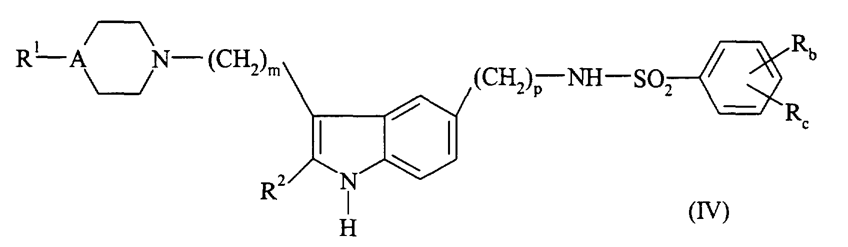 dans laquelle R¹, A, R², R_{b,} R_{c,} m et p ont la même signification que dans la formule (I),
- qui subit une condensation sur l'azote indolique avec de l'acrylonitrile, suivi d'une hydrolyse du dérivé nitrile en milieu alcalin pour conduire à un composé de formule (I/a), cas particulier de la formule (I): 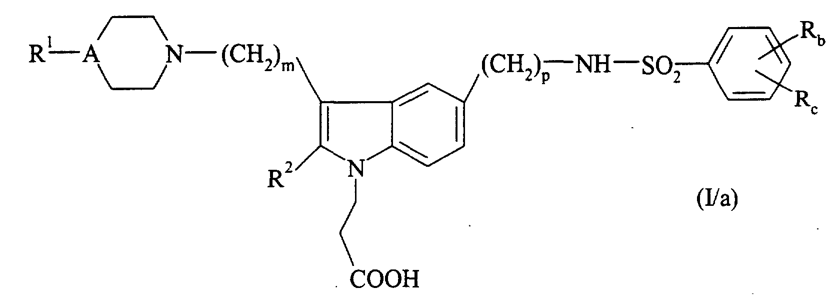 dans laquelle R¹, A, R², R_{b,} R_{c,} m et p ont la même signification que dans la formule (I),
- dont la fonction acide carboxylique est éventuellement transformée par réduction en aldéhyde, pour réagir avec un ylure de phosphore approprié, et qui après une réduction catalytique conduit à (I/b), cas particulier de la formule (I): 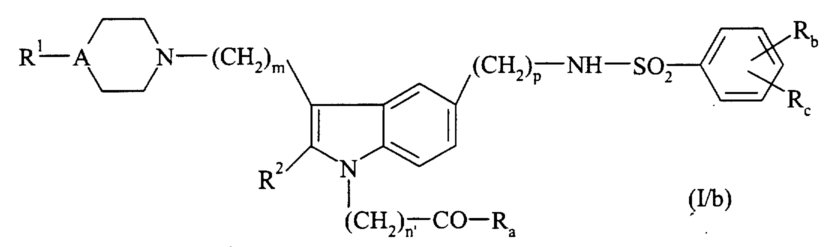 dans laquelle R¹, A, R², Rₐ, R_{b}, R_{c}, m et p ont la même signification que dans la formule (I), et n' est un entier compris entre 4 et 6,
composés de formule (I/b) qui peuvent être soumis à une hydrolyse de la fonction ester, en milieu basique ou acide selon les groupements réactifs sur la molécule, pour conduire au composé de formule (I/c) : 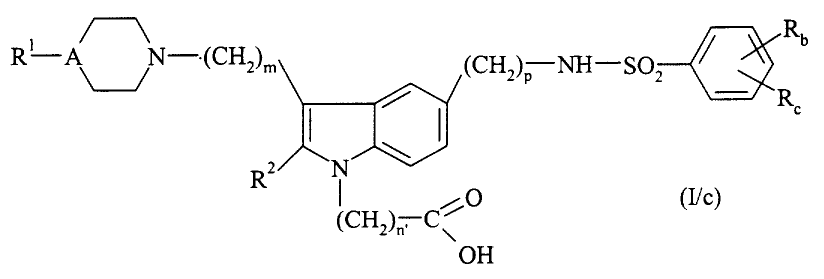 cas particulier des composés de fomule (I) dans laquelle R¹, R², R_{b}, R_{c}, m et p sont tels que définis dans la formule (I), et n' est un entier compris entre 4 et 6,
composés (I/a), (I/b) et (I/c) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable,
étant entendu qu'à tout moment jugé opportun au cours du procédé précédemment décrit, la fonction acide carboxylique peut être estérifiée, ou bien la fonction ester carboxylique peut être hydrolysée en acide correspondant, ce dernier pouvant être à nouveau transformé en un autre ester pour les besoins de la synthèse.

12. Compositions pharmaceutiques contenant comme principe actif au moins un composé selon l'une quelconque des revendications 1 à 10 seul ou en combinaison avec un ou plusieurs excipients ou véhicules inertes, non toxiques, pharmaceutiquement acceptables.

13. Compositions pharmaceutiques selon la revendication 12 contenant au moins un principe actif selon l'une quelconque des revendications 1 à 10 utiles pour la fabrication de médicaments utiles comme antagonistes des récepteurs du TXA₂ et des récepteurs 5-HT₂.

14. Compositions pharmaceutiques selon la revendication 12 contenant au moins un principe actif selon l'une quelconque des revendications 1 à 10 utiles pour la fabrication de médicaments utiles dans le traitement des maladies cardiovasculaires athéro-thrombotiques telles que l'infarctus du myocarde, l'angine de poitrine, les accidents vasculaires cérébraux, la maladie de Raynaud, ou encore de l'asthme, des brochospasmes, de la migraine et des maladies veineuses.
